(19) Europäisches Patentamt
European Patent Office
Office européen des brevets

(11) EP 1 621 184 A1

(12) DEMANDE DE BREVET EUROPEEN

(43) Date de publication:
**01.02.2006 Bulletin 2006/05**

(51) Int Cl.:
***A61K 8/87*** *(2006.01)* ***A61Q 5/10*** *(2006.01)*

(21) Numéro de dépôt: **05291419.9**

(22) Date de dépôt: **30.06.2005**

(84) Etats contractants désignés:
**AT BE BG CH CY CZ DE DK EE ES FI FR GB GR HU IE IS IT LI LT LU MC NL PL PT RO SE SI SK TR**
Etats d'extension désignés:
**AL BA HR LV MK YU**

(30) Priorité: **01.07.2004 FR 0451390**

(71) Demandeur: **L'OREAL**
**75008 Paris (FR)**

(72) Inventeurs:
- **Rollat-Corvol, Isabelle**
  **75017 Paris (FR)**
- **Gawtrey, Jonathan**
  **92100 Boulogne (FR)**

(74) Mandataire: **Fevrier, Murielle Françoise E.**
**L'OREAL**
**D.I.P.I.**
**25-29 Quai Aulagnier**
**F-92600 Asnieres (FR)**

# (54) Composition de coloration comprenant un polymère filmogène élastomère et une matière colorante

(57) L'invention a pour objet une composition de coloration comprenant, dans un milieu approprié à la coloration, au moins une matière colorante et au moins un polymère filmogène élastomère choisi de telle sorte que le film obtenu par séchage de ce polymère, à température ambiante et à un taux d'humidité relative de 55 %, présente un profil mécanique défini par au moins :

(a) un taux d'allongement à la rupture ($\varepsilon_r$) supérieur ou égal à 800 %,

(b) une recouvrance instantanée ($R_i$) au moins égale à 75 %, après un allongement de 150 %,

(c) une recouvrance ($R_{300}$) à 300 secondes supérieure à 80 %.

Cette composition permet notamment d'obtenir des colorations intenses, résistantes aux agents extérieurs.



Printed by Jouve, 75001 PARIS (FR)

## Description

L'invention a pour objet une composition de coloration comprenant, dans un milieu approprié à la coloration des matières kératiniques, une matière colorante et un polymère filmogène aux caractéristiques élastomères particulières, ainsi que le procédé de coloration des matières kératiniques, en particulier les fibres kératiniques, à partir de cette composition.

Depuis longtemps, de nombreuses personnes cherchent à modifier la couleur de leur peau, de leurs cils ou de leurs cheveux et en particulier à masquer leurs cheveux blancs. Pour ce faire, plusieurs technologies ont été développées.

Il est connu de teindre les fibres kératiniques et en particulier les cheveux humains, avec des compositions de teinture contenant des colorants directs. Les colorants classiques qui sont utilisés sont en particulier des colorants du type nitrés benzèniques, anthraquinoniques, nitropyridiniques, azoïques, azoïques, xanthéniques, acridiniques, aziniques, triarylméthane ou des colorants naturels. Ces colorants peuvent être non ioniques, anioniques, cationiques ou amphotères.

Ces colorants qui sont des molécules colorées et colorantes ayant une affinité pour les fibres sont appliqués sur les fibres kératiniques pendant un temps nécessaire à l'obtention de la coloration désirée, puis rincés.

Les colorations qui en résultent sont des colorations particulièrement chromatiques qui sont cependant temporaires ou semi-permanentes car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à la lumière du fait de la faible résistance du chromophore vis-à-vis des attaques photochimiques et conduisent dans le temps à un affadissement de la coloration des cheveux.

Il est déjà connu d'introduire dans les compositions de coloration directe des polymères classiquement utilisés pour la formulation des produits capillaires de coiffage. Ces polymères sont en général des polymères filmogènes anioniques, amphotères ou non ioniques, qui conduisent à la formation de films possédant un caractère plus ou moins dur et cassant.

Il existe un besoin dans le domaine de la coloration de composition permettant d'obtenir des colorations intenses, dans des nuances variées, qui résistent dans le temps, notamment aux agents extérieurs tels que la lumière, le shampooing, la sueur tout en préservant la qualité de la matière kératinique et en particulier la facilité de coiffage, notamment un bon démêlage, de la douceur et un aspect agréable, non collant lorsqu'on applique cette composition sur les fibres kératiniques, ainsi qu'une facilité d'emploi. Il existe aussi un besoin de disposer de compositions de coloration aptes à former sur ces matières kératiniques un film souple qui suit les mouvements de la peau ou de la chevelure, sans effet de tiraillement, de lourdeur, ou de sensation rigide.

Ce but est atteint par la présente invention qui concerne une composition de coloration comprenant, dans un milieu approprié à la coloration, au moins une matière colorante et au moins un polymère filmogène élastomère choisi de telle sorte que le film obtenu par séchage de ce polymère, à température ambiante et à un taux d'humidité relative de 55 %, présente un profil mécanique défini par au moins :

(a) un taux d'allongement à la rupture ($\varepsilon_r$) supérieur ou égal à 800 %,
(b) une recouvrance instantanée ($R_i$) au moins égale à 75 %, après un allongement de 150 %,
(c) une recouvrance ($R_{300}$) à 300 secondes supérieure à 80 %

Cette composition permet notamment d'obtenir des colorations intenses, résistantes aux agents extérieurs tout en préservant l'intégrité des matières kératiniques et un bon coiffage par la formation d'un film souple, non cassant sur les matières kératiniques humaines, et qui suit leurs mouvements.

Par "au moins un" polymère filmogène élastomère, on entend au sens de l'invention, un ou plusieurs (2, 3 ou plus) polymères filmogènes élastomères.

Par matière colorante, on entend toute molécule colorée qui lorsqu'elle est mise en contact avec la matière kératinique colore cette matière ou toute molécule non colorée qui en contact avec la matière kératinique colore la matière kératinique sans l'aide d'agent chimique additionel, en particulier sans l'aide d'un agent oxydant. De préférence, les matières colorantes selon l'invention sont des matières colorées, c'est-à-dire des molécules colorées qui, lorsqu'elles sont en contact avec la matière kératinique, colorent cette matière kératinique.

Un autre objet de la présente invention se rapporte à un procédé de coloration des matières kératiniques, en particulier les fibres kératiniques à partir de la composition de l'invention.

Encore un objet de la présente invention se rapporte à l'utilisation de cette composition pour la coloration des matières kératiniques, en particulier les fibres kératiniques telles que les cheveux.

Au sens de la présente invention, on entend par film obtenu par séchage à température ambiante (22°C ± 2°C) et à un taux d'humidité relative de 55 % ± 5%, le film obtenu dans ces conditions à partir d'un mélange à 6 % de

matière active (m.a.) de polymère filmogène élastomère dans un mélange 30 % en poids d'éthanol et de 70 % en poids d'eau, par rapport au poids total alcool+eau, la quantité de mélange étant adaptée pour obtenir dans une matrice en téflon, un film d'épaisseur de 500 μm ± 50 μm. Le séchage est poursuivi jusqu'à ce que le poids du film n'évolue plus, ce qui représente environ 12 jours. Les polymères filmogène solubles ou partiellement solubles dans l'éthanol sont testés dans l'éthanol seul. Les autres polymères sont testés dans l'eau seule, sous forme soluble ou dispersée.

Au sens de la présente invention, le taux d'élongation à la rupture et le taux de recouvrance sont évalués aux moyens des essais décrits ci-après.

Pour effectuer les essais de traction, le film est découpé en éprouvettes de forme rectangulaire, de longueur 80 mm et de largeur 15 mm.

Les essais sont réalisés sur un appareil commercialisé sous l'appellation Lloyd ou commercialisé sous l'appellation Zwick dans les mêmes conditions de températures et d'humidité que pour le séchage, c'est-à-dire une température de 22°C ± 2 °C et un taux d'humidité relative de 50 % ± 5 %.

Les éprouvettes sont étirées à la vitesse de 20mm/min et la distance entre les mors est de 50 ± 1 mm.

Pour déterminer la recouvrance instantanée ($R_i$), on procède comme suit :

- on étire l'éprouvette de 150 % ($\varepsilon_{max}$) c'est-à-dire 1,5 fois sa longueur initiale ($l_0$),
- on relâche la contrainte en imposant une vitesse de retour égale à la vitesse de traction, soit 20mm/min, et on mesure l'allongement de l'éprouvette en pourcentage, après retour à charge nulle ($\varepsilon_i$).

La recouvrance instantanée en % ($R_i$) est donnée par la formule ci-après :

$$R_i = ((\varepsilon_{max} - \varepsilon_i)/ \varepsilon_{max}) \times 100$$

Pour déterminer la recouvrance à 300 secondes, on maintient à contrainte nulle pendant 300 secondes supplémentaires, l'éprouvette ayant subi les opérations précédentes, et on mesure son taux d'allongement en pourcentage ($\varepsilon_{300s}$).

La recouvrance à 300 secondes en % ($R_{300s}$) est donnée par la formule ci-après :

$$R_{300s} = ((\varepsilon_{max} - \varepsilon_{300s})/ \varepsilon_{max}) \times 100$$

De façon avantageuse, le ou les polymères de la composition selon l'invention, éventuellement associé(s) à un agent auxiliaire de filmification, sont tels qu'ils forment, dans les conditions des tests ci-dessus, un film d'allongement à la rupture allant de 800 % à 3000 % ; de recouvrance instantanée de 75 % à 100 % ; et une recouvrance à 300 secondes allant de 85 % à 100 %.

Dans les compositions conformes à l'invention, le polymère filmogène élastomère ou le mélange de polymères filmogènes élastomères est, de préférence, présent à une concentration de 0,05 % à 20 % en poids, plus préférentiellement de 0,1 % à 15 % en poids, et par exemple de 0,25 % à 10 % en poids par rapport au poids total de la composition.

De manière avantageuse, le polymère filmogène élastomère est choisi dans le groupe comprenant les polyuréthannes, les alcools polyvinyliques, les polymères comprenant au moins un motif (méth)acrylique, leurs associations. Il peut se présenter sous forme d'homopolymère ou de copolymère. En particulier, il se présente sous forme non réticulée dans la composition.

De préférence, le (ou les) polymères filmogènes élastomères utiles dans la présente invention sont solubles ou hydrodispersibles en milieu aqueux ou hydroalcoolique. En particulier, le ou les polymères filmogènes élastomères sont solubles à au moins 10 g en matière active dans 90 g de milieu aqueux ou hydroalcoolique (contenant 70 % eau et 30 % éthanol), à température ambiante et pression atmosphérique.

Selon un mode de réalisation particulier, le film formé à partir de la composition de l'invention présente une faible sensibilité à l'eau, par exemple en atmosphère d'humidité relative de 30 à 80%, c'est-à-dire que le film garde ses propriétés élastomères pendant plusieurs heures. Il est souple, non cassant et suit bien les mouvements de la peau et/ou de la chevelure. En particulier, entre 30 % et 80 % d'humidité relative, le taux d'allongement à la rupture du film obtenu ne varie pas de plus de 50 % (± 400 %) et/ou sa recouvrance instantanée ne varie pas de plus de 25 % (18,75 %). Autrement dit, entre 30 et 80 % d'humidité relative, le taux d'allongement à la rupture du film obtenu est compris entre 400 % et 1200 % et/ou sa recouvrance instantanée est comprise entre 57 et 93 %.

Les polymères filmogènes élastomères utiles dans la composition de l'invention sont connus de la technique.

Ils peuvent par exemple être synthétisés selon la méthode décrite dans la demande de brevet FR 2 815 350.

A titre de matière colorante, on peut citer les colorants directs éventuellement fluorescents , les pigments, la DHA (dihydroxyacétone) et les dérivés de DHA.

Les colorants directs sont des colorants solubles dans l'eau ou dans un milieu solvant. A titre de colorant direct, on peut citer les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

Parmi les colorants directs benzéniques, on peut citer de manière non limitative les composés suivants:

- 1,4-diamino-2-nitrobenzène
- 1-amino-2 nitro-4-β- hydroxyéthylaminobenzène
- 1-amino-2 nitro-4-bis(β-hydroxyéthyl)-aminobenzène
- 1,4-Bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-Diamino-4-nitrobenzène
- 1-amino-2-β-hydroxyéthylamino-5-nitrobenzène
- 1,2-Bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,β-dihydroxypropyloxy-3-méthylamino-4-nitrobenzène
- 1-βhydroxyéthylamino-4-β,β-dihydroxypropyloxy-2-nitrobenzène
- 1-β,β-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, on peut citer les colorants azoïques cationiques décrits dans les demandes de brevets WO 95/15144, WO-95/01772 et EP-714954 dont le contenu fait partie intégrante de l'invention.

Parmi ces composés, on peut tout particulièrement citer les colorants suivants:

- chlorure de 1,3-diméthyl-2-[[4-(diméthylamino)phényl]azo]-1H-Imidazolium,
- chlorure de 1,3-diméthyl-2-[(4-aminophényl)azo]-1H-Imidazolium,
- méthylsulfate de 1-méthyl-4-[(méthylphénylhydrazono)méthyl]-pyridinium.

On peut également citer parmi les colorants directs azoïques les colorants suivants, décrits dans le COLOUR INDEX INTERNATIONAL 3e édition :

- Disperse Red 17
- Acid Yellow 9
- Acid Black 1
- Basic Red 22
- Basic Red 76

- Basic Yellow 57
- Basic Brown 16
- Acid Yellow 36
- Acid Orange 7
- Acid Red 33
- Acid Red 35
- Basic Brown 17
- Acid Yellow 23
- Acid Orange 24
- Disperse Black 9.

On peut aussi citer le 1-(4'-aminodiphénylazo)-2-méthyl-4bis-(β-hydroxyéthyl) aminobenzène et l'acide 4-hydroxy-3-(2-méthoxyphénylazo)-1-naphtalène sulfonique.

Parmi les colorants directs quinoniques, on peut citer les colorants suivants :

- Disperse Red 15
- Solvent Violet 13
- Acid Violet 43
- Disperse Violet 1
- Disperse Violet 4
- Disperse Blue 1
- Disperse Violet 8
- Disperse Blue 3
- Disperse Red 11
- Acid Blue 62
- Disperse Blue 7
- Basic Blue 22
- Disperse Violet 15
- Basic Blue 99

ainsi que les composés suivants :

- 1-N-méthylmorpholiniumpropylamino-4-hydroxyanthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β-γ-dihydroxypropylamino)-anthraquinone.

Parmi les colorants aziniques, on peut citer les composés suivants :

- Basic Blue 17
- Basic Red 2.

Parmi les colorants triarylméthaniques, on peut citer les composés suivants :

- Basic Green 1
- Acid blue 9
- Basic Violet 3
- Basic Violet 14
- Basic Blue 7
- Acid Violet 49
- Basic Blue 26
- Acid Blue 7

Parmi les colorants indoaminiques, on peut citer les composés suivants :

- 2-β-hydroxyéthlyamino-5-[bis-(β-4'-hydroxyéthyl)amino]anilino-1,4-benzoquinone

- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine. On peut également utiliser les extraits ou décoctions contenant ces colorants naturels et notamment les cataplasmes ou extraits à base de henné.

Les colorants directs utiles dans la présente invention peuvent aussi être des colorants fluorescents. Par colorant fluorescent, on entend au sens de la présente invention un colorant qui est une molécule qui colore par elle-même et donc absorbe la lumière du spectre visible et éventuellement de l'ultraviolet (longueurs d'onde allant de 360 à 760 nanomètres) mais qui, contrairement à un colorant classique, transforme l'énergie absorbée en lumière fluorescente de plus grande longueur d'onde émise dans la partie visible du spectre.

Le colorant fluorescent utile dans la présente invention est à différencier d'un agent éclaircissant optique. Les agents éclaircissants optiques appelés généralement azurants optiques, ou "brighteners", ou "fluorescent brighteners", ou "fluorescent brightening agents", ou "fluorescent whitening agents", ou "whiteners", ou encore "fluorescent whiteners" en terminologie anglo-saxone, sont des composés transparents incolores. Ces composés ne colorent pas car ils n'absorbent pas dans la lumière visible, mais uniquement dans les ultraviolets (longueurs d'onde allant de 200 à 400 nanomètres). Ils transforment l'énergie absorbée en lumière fluorescente de plus grande longueur d'onde émise dans la partie visible du spectre ; l'impression de couleur est alors uniquement engendrée par la lumière purement fluorescente à prédominante bleue (longueurs d'onde allant de 400 à 500 nanomètres).

Le colorant fluorescent mis en oeuvre peut être soluble dans le milieu de la composition ou non soluble comme les pigments fluorescents.

A titre d'exemples de colorants fluorescents susceptibles d'être mis en oeuvre, on peut citer les colorants fluorescents appartenant aux familles suivantes : les naphtalimides ; les coumarines cationiques ou non ; les xanthénodiquinolizines (comme notamment les sulforhodamines) ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges, de préférence aux familles suivantes : les naphtalimides ; les coumarines cationiques ou non ; les azaxanthènes ; les naphtolactames ; les azlactones ; les oxazines ; les thiazines ; les dioxazines ; les colorants fluorescents polycationiques de type azoïque, azométhinique, ou méthinique, seuls ou en mélanges.

Parmi les colorants fluorescents solubles, on peut notamment citer:

- le Jaune Brilliant B6GL commercialisé par la société SANDOZ et de structure suivante :

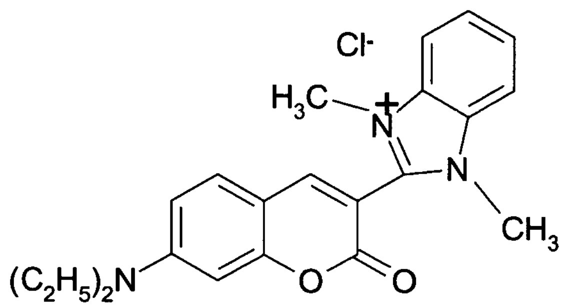

(F1)

- le Basic Yellow 2, ou Auramine O commercialisé par les sociétés PROLABO, ALDRICH ou CARLO ERBA et de structure suivante :

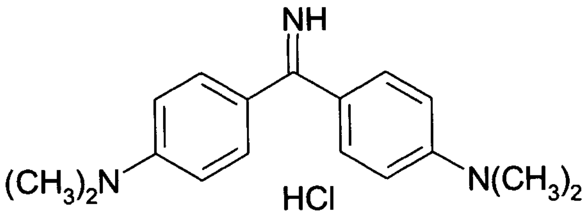

(F2)

monochlorhydrate de 4,4'-(imidocarbonyl)bis(N,N-diméthylaniline) - CAS number 2465-27-2.

La composition de l'invention peut comprendre des pigments. Dans le cadre de l'invention, on entend par pigment toute entité organique et / ou minérale dont la solubilité dans l'eau est inférieure à 0,01 % à 20 °C, de préférence inférieure à 0,001 %, et présentant une absorption entre 350 et 700 nm, de préférence une absorption avec un maximum.

Les pigments utiles dans la présente invention sont choisis parmi tous les pigments organiques et / ou minéraux connus de la technique, notamment ceux qui sont décrits dans l'encyclopédie de technologie chimique de Kirk-Othmer et dans l'encyclopédie de chimie industrielle de Ullmann ou dans « International Cosmetic Ingredient Dictionnary and Handbook », édition 1997, pages 371 à 386 et 524 à 528 publié par « The cosmetic, Toiletry and fragrance association ».

Ces pigments peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les références CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :

- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant :

- un noyau inorganique,
- au moins un liant assurant la fixation des pigments organiques sur le noyau, et
- au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. On peut aussi citer à titre de nacre des particules irisées produites par certains mollusques dans leur coquille,.

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" *Journal of phisical chemistry B, vol 101, 1997, pp* 9463-9475. et Peng, Xiaogang et al, "Epitaxial Growth of highly Luminescent CdSe/CdS core/shell nanocrystals with photostability and electronic accessibility" *Journal of the American Chemical Society, vol 119, N°30, pp 7019-7029.*

La variété des pigments qui peuvent être utilisés dans la présente invention permet d'obtenir une riche palette de couleurs, ainsi que des effets optiques particuliers tels que des effets métalliques, interférentiels.

La taille d'un pigment autre que les nacres en solution est généralement comprise entre 10 nm et 10 μm, de préférence entre 50 nm et 5 μm, et plus préférentiellement entre 100 nm et 3 μm. La taille d'une nacre en solution est généralement comprise entre 1 et 200 μm, de préférence entre 1 et 80 μm, et plus préférentiellement entre 1 et 50 μm.

Le ou les pigments sont chacun généralement présents dans la composition conforme à l'invention dans des quantités généralement comprises entre 0,05 et 40 % du poids total de la composition, de préférence de 0,1 à 35 %.

A titre de matière colorante, on peut aussi citer la dihydroxyacétone ou ses dérivés (DHA par la suite dans le texte). La DHA est connue depuis lontemps dans la coloration des matières kératiniqes (Bobin et al. J. Soc. Cosmet. Chem., 35 pages 265-272, 1984). La DHA réagit avec les acides aminés naturellement contenus dans la matière kératinique et par une réaction de Maillard forme des métanoïdes (Maillard L.C., C.R. Acad. Sci. 154, 66-68, 1912). A titre de composés utiles dans invention, on peut citer la DHA ainsi que ses dérivés tels que décrits dans la demande GB 953 170, WO 96/09807, FR-A-2597345.

Le ou les matières colorantes représentent de préférence de 0,001 à 20% en poids environ du poids total de la composition prête à l'emploi et encore plus préférentiellement de 0,005 à 10% en poids environ.

La composition de la présente invention peut de plus contenir des bases d'oxydations et des coupleurs classiquement utilisés pour la coloration par oxydation.

A titre d'exemple, on peut citer les paraphénylènediamines, les bis-phénylalkylènediamines, les para-aminophénols, les ortho-aminophénols, les bases hétérocycliques et leurs sels d'addition.

Les coupleurs sont par exemple les coupleurs métaphénylènediamines, les coupleurs méta-aminophénols, les coupleurs métadiphénols, les coupleurs naphtaléniques, les coupleurs hétérocycliques et leur sels d'addition.

Lorsqu'ils sont présents, les bases et les coupleurs sont chacun généralement présents en quantité comprise entre 0,001 et 10 % en poids environ du poids total de la composition tinctoriale, de préférence entre 0,005 et 6 %.

La composition peut, en outre comprendre un agent auxiliaire de filmification tel qu'un agent plastifiant et/ou un agent facilitant la filmification du ou des polymères élastomères sur les matières kératiniques, et dont la fonction est de modifier les propriétés du ou des polymères élastomères. Un tel agent auxiliaire de filmification peut être choisi parmi

tous les composés connus de l'homme du métier comme étant susceptibles de remplir la fonction recherchée, et être notamment choisi parmi les agents plastifiants et les agents de coalescence. Le ou les polymères filmogènes élastomères, éventuellement associés à un agent plastifiant et/ou un agent facilitant la filmification sont aptes à former un film, après évaporation du milieu cosmétique. Cette évaporation peut être faite à l'air libre ou en apportant de la chaleur, par exemple à l'aide d'un séchoir.

Comme exemple d'agent plastifiant et/ou facilitant la filmification sur les matières kératiniques, on peut utiliser ceux décrits dans le document FR-A-2 782 917. En particulier, cet agent est choisi parmi les plastifiants ou agents de coalescence usuels, tels que:

- les glycols et leurs dérivés tels que le diéthylène glycol éthyléther, le diéthylène glycol méthyléther, le diéthylène glycol butyléther ou encore le diéthylène glycol hexyléther, l'éthylène glycol éthyléther, l'éthylène glycol butyléther, l'éthylène glycol hexyléther, le pentylène glycol,
- les esters de glycérol,
- les dérivés de propylène glycol et en particulier le propylène glycol phényléther, le propylène glycol diacétate, le dipropylène glycol butyléther, le tripropylène glycol butyléther, le propylène glycol méthyléther, le dipropylène glycol éthyléther, le tripropylène glycol méthyléther et le diéthylène glycol méthyléther, le propylène glycol butyléther,
- les esters d'acides notamment carboxyliques, tels que des citrates, des phtalates, des adipates, des carbonates, des tartrates, des phosphates, des sébaçates,
- leurs mélanges.

La quantité d'agent plastifiant et/ou d'agent de filmification peut être choisie par l'homme du métier sur base de ses connaissances générales, de manière à obtenir un système polymérique (polymères élastomères + agent plastifiant et/ou agent de filmification) conduisant à un film ayant les propriétés mécaniques souhaitées, tout en conservant à la composition les propriétés cosmétiques recherchées. En pratique, cette quantité varie de 0,01 % à 25 % du poids total de la composition et mieux de 0,01 % à 15 %.

Le milieu approprié pour la teinture appelé aussi support de teinture est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C1-C4, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

Pour la teinture des fibres kératiniques humaines, le milieu de coloration est un milieu cosmétique approprié.

Les solvants peuvent être présents dans des proportions de préférence comprises entre 1 et 40 % en poids environ par rapport au poids total de la composition tinctoriale, et encore plus préférentiellement entre 5 et 30 % en poids environ.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes autres que ceux de l'invention et en particulière des polymères fixants non ioniques, cationiques, anioniques, amphotères, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

Le pH de la composition tinctoriale conforme à l'invention est généralement compris entre 3 et 12 environ, et de préférence entre 5 et 11 environ.

Il peut être ajusté à la valeur désirée au moyen d'agents acidifiants ou alcalinisants habituellement utilisés en teinture des fibres kératiniques ou bien encore à l'aide de systèmes tampons classiques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants, on peut citer, à titre d'exemple, l'ammoniaque, les carbonates alcalins, les alcanolamines telles que les mono-, di- et triéthanolamines ainsi que leurs dérivés, les hydroxydes de sodium ou de

potassium et les composés de formule (III) suivante :

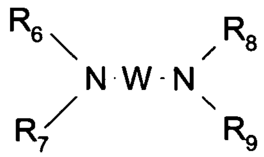

(III)

dans laquelle W est un reste propylène éventuellement substitué par un groupement hydroxyle ou un radical alkyle en C1-C4 ; R6, R7, R8 et R9, identiques ou différents, représentent un atome d'hydrogène, un radical alkyle en C1-C4 ou hydroxyalkyle en C1-C4.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a aussi pour objet un procédé de teinture directe qui comprend l'application de la composition de l'invention sur les fibres kératiniques. Après un temps de pause, les fibres kératiniques sont rincées laissant apparaître des fibres colorées, non collantes et faciles à coiffer ou à mettre en forme. Le temps de pause est généralement compris entre 3 à 50 minutes environ, de préférence 5 à 30 minutes environ.

Lorsque la composition tinctoriale comprend une base d'oxydation et/ou un coupleur, la composition tinctoriale peut alors contenir un agent oxydant. Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

L'agent oxydant peut être ajouté à la composition de l'invention juste au moment de l'emploi ou il peut être mis en oeuvre à partir d'une composition oxydante le contenant, appliquée simultanément ou séquentiellement à la composition de l'invention. La composition oxydante peut également renfermer divers adjuvants utilisés classiquement dans les compositions pour la teinture des cheveux et tels que définis précédemment.

Le pH de la composition oxydante renfermant l'agent oxydant est tel qu'après mélange avec la composition tinctoriale, le pH de la composition résultante appliquée sur les fibres kératiniques varie de préférence entre 3 et 12 environ, et encore plus préférentiellement entre 5 et 11. Il peut être ajusté à la valeur désirée au moyen d'agents acidifiants ou alcalinisants habituellement utilisés en teinture des fibres kératiniques et tels que définis précédemment.

La composition qui est finalement appliquée sur les fibres kératiniques peut se présenter sous des formes diverses, telles que sous forme de liquides, de crèmes, de gels ou sous toute autre forme appropriée pour réaliser une teinture des fibres kératiniques, et notamment des cheveux humains.

**EXEMPLES**

Les quantités sont données en pourcentage massique et M.A. signifie matière active.

**Exemple 1 :**

On prépare une composition de teinture qui comprend :

| | |
|---|---|
| Polyuréthane (NMDEA[1) / PTMO 2900[2) / IPDI[3) - 3/ 1/ 4) | 2 % MA |
| Basic Red 51 | 0,15 g |
| Ethanol | 5 g |
| 2-amino 2-méthyl 1 propanol | Qs pH 8.5 |
| Eau démineralisée | Qsp 100g |
| [1) - N-méthyldiéthanolamine<br>[2) - Poly (tétraméthylène oxyde) ayant une masse moyenne en poids de 2900<br>[3) - Isophoronediisocianate | |

Le polymère a été préparé selon la méthode de synthèse décrite dans la demande de brevet FR 2 815 350.

Le copolymère de polyuréthane présente les caractéristiques suivantes, mesurées comme ci-dessus :

- $\varepsilon_r$ = 1500 %
- $R_i$ = 82 %
- $R_{300}$ = 92 %
- Soluble dans l'eau à au moins 10 g par litre

Cette composition appliquée 30 minutes sur des cheveux châtains, puis rincée, confére après séchage un effet rouge tenace.

**Revendications**

**1.** Composition de coloration comprenant, dans un milieu approprié à la coloration, au moins une matière colorante et au moins un polymère filmogène élastomère choisi de telle sorte que le film obtenu par séchage de ce polymère, à température ambiante et à un taux d'humidité relative de 55 %, présente un profil mécanique défini par au moins :

(a) un taux d'allongement à la rupture ($\varepsilon_r$) supérieur ou égal à 800 %,
(b) une recouvrance instantanée ($R_i$) au moins égale à 75 %, après un allongement de 150 %,
(c) une recouvrance ($R_{300}$) à 300 secondes supérieure à 80 %.

**2.** Composition selon la revendication 1, **caractérisée en ce que** le polymère filmogène élastomère est soluble dans un milieu aqueux ou hydroalcoolique.

**3.** Composition selon la revendication 1 ou 2, **caractérisée en ce qu'**entre 30 % et 80 % d'humidité relative, le taux d'allongement à la rupture du film obtenu est comprise entre 400 % et 1200 % et/ou sa recouvrance instantanée est comprise entre 57 et 93 %.

**4.** Composition selon l'une quelconque des revendications précédentes, **caractérisée en ce que** le polymère filmogène élastomère ou le mélange de polymères filmogènes élastomères est présent à une concentration de 0,05 % à 20 % en poids, plus préférentiellement de 0,1 % à 15 % en poids.

**5.** Composition selon l'une quelconque des revendications précédentes, **caractérisée en ce que** le polymère filmogène est choisi dans le groupe comprenant les polyuréthannes, les alcools polyvinyliques, les polymères comprenant au moins un motif (méth)acrylique, leurs associations.

**6.** Composition selon l'une quelconque des revendications précédentes dans laquelle la ou les matières colorantes sont choisies parmi les colorants directs, les colorants directs fluorescents, les pigments, la DHA et ses dérivés.

**7.** Composition selon la revendication 6 dans laquelle le ou les colorants directs sont choisis parmi les colorants directs nitrés benzéniques neutres, acides ou cationiques, les colorants directs azoïques neutres acides ou cationiques, les colorants directs quinoniques et en particulier anthraquinoniques neutres, acides ou cationiques, les colorants directs aziniques, les colorants directs triarylméthaniques, les colorants directs indoaminiques et les colorants directs naturels.

**8.** Composition selon la revendication 6 dans laquelle les colorants directs sont des colorants fluorescents.

**9.** Composition selon la revendication 6 dans laquelle le ou les pigments sont choisis parmi les pigments blancs ou colorés, les laques, les pigments à effets spéciaux.

**10.** Composition selon l'une quelconque des revendications précédentes dans laquelle la quantité de matière colorante est comprise entre 0,001 à 20% en poids environ du poids total de la composition.

**11.** Procédé de coloration des matières kératiniques qui comprend l'application sur ia matière kératinique d'une composition telle que définie aux revendications 1 à 10 pendant un temps suffisant pour colorer les matières kératiniques.

**12.** Procédé selon la revendication 10 pour la coloration des fibres kératiniques.

**13.** Utilisation d'une composition selon l'une quelconque des revendications 1 à 10 pour la teinture des matières kératiniques

**14.** Utilisation selon la revendication 13 pour la coloration des fibres kératiniques.

Office européen des brevets

# RAPPORT DE RECHERCHE EUROPEENNE

Numéro de la demande
EP 05 29 1419

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| X | FR 2 815 350 A (OREAL) 19 avril 2002 (2002-04-19) * le document en entier * | 1-14 | A61K7/13 |
| X | FR 2 786 391 A (OREAL) 2 juin 2000 (2000-06-02) * revendications 1,8 * | 1-14 | |
| X | FR 2 786 392 A (OREAL) 2 juin 2000 (2000-06-02) * revendications 1,7 * | 1-14 | |
| X | FR 2 795 634 A (OREAL) 5 janvier 2001 (2001-01-05) * page 28, ligne 32-36; revendications 1,34; exemples 1,2 * | 1-14 | |

DOMAINES TECHNIQUES RECHERCHES (Int.Cl.7)

A61K

Le présent rapport a été établi pour toutes les revendications

1

| Lieu de la recherche | Date d'achèvement de la recherche | Examinateur |
|---|---|---|
| Munich | 16 septembre 2005 | Yon, J-M |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
..................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 05 29 1419

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-09-2005

| Document brevet cité au rapport de recherche | | Date de publication | Membre(s) de la famille de brevet(s) | | | Date de publication |
|---|---|---|---|---|---|---|
| FR 2815350 | A | 19-04-2002 | AU | 9569201 | A | 29-04-2002 |
| | | | EP | 1326908 | A1 | 16-07-2003 |
| | | | WO | 0232978 | A1 | 25-04-2002 |
| | | | JP | 2004511637 | T | 15-04-2004 |
| | | | US | 2004052753 | A1 | 18-03-2004 |
| FR 2786391 | A | 02-06-2000 | AU | 745846 | B2 | 11-04-2002 |
| | | | AU | 1276700 | A | 13-06-2000 |
| | | | BR | 9907723 | A | 17-10-2000 |
| | | | CN | 1295459 | A | 16-05-2001 |
| | | | EP | 1051146 | A1 | 15-11-2000 |
| | | | WO | 0030594 | A1 | 02-06-2000 |
| | | | JP | 2002530310 | T | 17-09-2002 |
| | | | JP | 2004307516 | A | 04-11-2004 |
| | | | PL | 341980 | A1 | 07-05-2001 |
| | | | RU | 2183449 | C2 | 20-06-2002 |
| | | | US | 6391292 | B1 | 21-05-2002 |
| FR 2786392 | A | 02-06-2000 | AU | 746140 | B2 | 18-04-2002 |
| | | | AU | 1276600 | A | 13-06-2000 |
| | | | BR | 9907724 | A | 17-10-2000 |
| | | | CN | 1295458 | A | 16-05-2001 |
| | | | EP | 1051145 | A1 | 15-11-2000 |
| | | | WO | 0030593 | A1 | 02-06-2000 |
| | | | JP | 2002530309 | T | 17-09-2002 |
| | | | PL | 341932 | A1 | 07-05-2001 |
| | | | RU | 2200534 | C2 | 20-03-2003 |
| | | | US | 6497864 | B1 | 24-12-2002 |
| FR 2795634 | A | 05-01-2001 | AT | 235878 | T | 15-04-2003 |
| | | | BR | 0006944 | A | 31-07-2001 |
| | | | CA | 2341591 | A1 | 11-01-2001 |
| | | | CN | 1321079 | A | 07-11-2001 |
| | | | DE | 60001874 | D1 | 08-05-2003 |
| | | | DE | 60001874 | T2 | 18-12-2003 |
| | | | EP | 1064920 | A1 | 03-01-2001 |
| | | | ES | 2195843 | T3 | 16-12-2003 |
| | | | WO | 0101936 | A1 | 11-01-2001 |
| | | | JP | 2001031539 | A | 06-02-2001 |
| | | | MX | PA01001630 | A | 08-04-2002 |
| | | | US | 6482400 | B1 | 19-11-2002 |

EPO FORM P0460

Pour tout renseignement concernant cette annexe : voir Journal Officiel de l'Office européen des brevets, No.12/82